# EUROPEAN PATENT APPLICATION

(11) **EP 2 759 299 A1**
(43) Date of publication of application: **30.07.2014**
(21) Application number: 13305034.4
(22) Date of filing: 14.01.2013
(51) Int. Cl.: A61K 31/715, A61K 31/726, A61P 37/00, A61P 1/00

(54) **Galactosaminogalactan comprising alpha 1-4 linked galactose and alpha 1-4 linked N-acetylgalactosamine for use in the treatment of at least one inflammatory disease**

(71) Applicant: INSTITUT PASTEUR, 75015 Paris (FR)
(72) Inventor: The designation of the inventor has not yet been filed
(74) Representative: Regimbeau

(57) **Abstract**

A first object of the invention is galactosaminogalactan comprising α1-4 linked galactose and α1-4 linked N-acetylgalactosamine for use in the treatment of at least one inflammatory disease. Another objet of the invention is a pharmaceutical composition comprising a galactosaminogalactan comprising α1-4 linked galactose and α1-4 linked N-acetylgalactosamine as defined in any of the above-cited claims, and optionally a pharmaceutically acceptable carrier. Another object of the invention is a pharmaceutical composition according to the invention, for use in the treatment of at least one inflammatory disease. Another object of the invention is an inhibitor of IL-1 RA for use in the treatment of human fungal diseases. Another object of the invention is a pharmaceutical composition comprising an inhibitor of IL-1 RA, and optionally a pharmaceutically acceptable carrier.

## Description

Inflammation is the body's defense reaction to injuries such as those caused by mechanical damage, infection or antigenic stimulation. An inflammatory reaction may be expressed pathologically when inflammation is induced by an inappropriate stimulus such as an autoantigen, is expressed in an exaggerated manner or persists well after the removal of the injurious agents.

While the etiology of inflammation is poorly understood, considerable information has been gained regarding the molecular aspects of inflammation (see for example Goldsby RA, Kindt TK, Osborne BA and Kuby J (2003) Immunology, 5th Edition, W.H. Freeman and Company, or Janeway CA, Travers P, Walport M, and Shlomchik M (2001) Immunobiology, 6th Edition, Garland Publishing).

Interleukin (IL)-1 was first cloned in the 1980s, and rapidly emerged as a key player in the regulation of inflammatory processes (see for reference Gabay et al., Nat Rev Rheumatol, 6: 232-241; 2010). The term IL-1 refers to two cytokines, IL-1α and IL-1β, which are encoded by two separate genes. The effects of IL-1 are tightly controlled by several naturally occurring inhibitors, such as IL-1 receptor antagonist (IL-1 RA), IL-1 receptor type II (IL-1RII), and other soluble receptors.

The role of the potent proinflammatory cytokine IL-1 in disease has been shown in a broad range of diseases, including rheumatoid arthritis and autoimmune diseases. IL-1 has also been implicated in inflammatory bowel diseases (IBD). Indeed, an imbalance between proinflammatory and antiinflammatory cytokines was found for the IL-1/IL-1 RA ratio in the inflamed mucosa of patients with Crohn's disease, ulcerative colitis, diverticulitis, and infectious colitis (Rogler et al.; World J Surg.;22(4):382-9; 1998).

Nowadays, blocking of IL-1 activity (especially IL-1β) is a standard therapy for patients with autoimmune diseases or lymphomas. Recombinant IL-1 RA, a potent Il-1 receptor antagonist, is approved for instance as a therapy for patients with rheumatoid arthritis, because it reduces symptoms of rheumatoid arthritis and slows the progressive joint destruction. It has also been subscribed to patients with smoldering/indolent myeloma with a high risk of progression to multiple myeloma.

The use of IL-1 receptor antagonists has been uniformly associated with beneficial effects in patients with hereditary autoinflammatory conditions associated with excessive IL-1 receptor activity (IL-1 signaling), either due to elevated Il-1 IL-1 RA deficiency. Successful treatment with IL-1 blockers has also been reported in other hereditary autoinflammatory diseases, as well as in nonhereditary inflammatory diseases, such as Schnizler syndrome, systemic-onset juvenile idiopathic arthritis and adult Still disease. The role of microcrystals in the regulation of IL-1β processing and release has provided the rationale for the use of IL-1 inhibitors in crystal-induced arthritis. Finally, preliminary results indicating that IL-1 targeting is efficacious in type 2 diabetes and smoldering myeloma have further broadened the spectrum of IL-1-driven diseases.

Recombinant Il-1 RA is however not devoid of sides effects, such as nausea (8%), diarrhea (7%), sinusitis (7%), flu-like syndrome (6%). The major side effect of recombinant Il-1 RA is the risk of infections (40%, severe in 2%), more particularly of upper respiratory tract (13%). Moreover, recent cases of hypersensitivity to recombinant Il-1 RA have been observed (Desai et al., Ann Pharmacother. ; 43(5): 967-972.; 2009).

There is therefore a need for alternative therapeutic compounds for the treatment for inflammatory diseases, in particular for inflammatory interleukin-1 mediated disease.

### FIGURE LEGEND

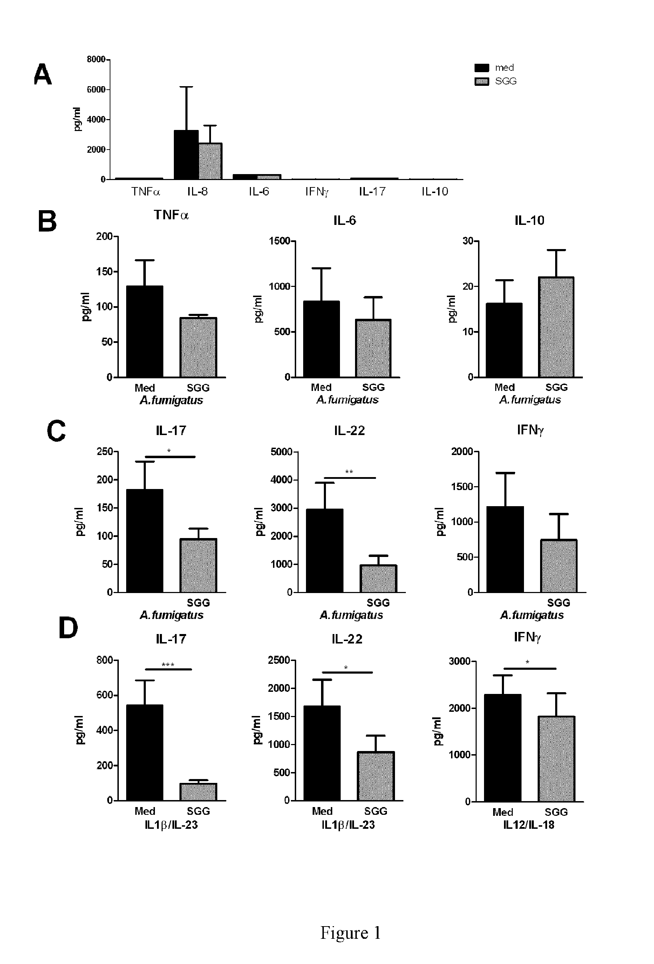

Figure 1: Induction of IL-1 RA by soluble galactosaminogalactan (SGG) is dependent on Syk and TLR3/TRIF. TNFα, IL-6, IL-8 and IL-10 concentrations in culture supernatants of human PBMCs stimulated for 24hours with 10 µg/ml SGG and IFNγ and IL-17 concentrations after 7 days of stimulation (b) TNFα, IL-6 and IL-10 concentrations in culture supernatants of human PBMCs (n=6 donors) stimulated for 24 hours with heat inactivated *A. fumigatus* conidia (1x107/ml) in the presence or absence of 10 µg/ml SGG. (c,d,e) IL-17, IL-22 and IFNγ concentrations in culture supernatants of human PBMCs stimulated for 7 days with heat inactivated *A. fumigatus* conidia (1x107/ml) (n=10 donors for IL-17 and IL-22, n=6 donors for IFNγ) (c), IL-1β/IL-23 (50/100 ng/ml) (n=14 donors) or IL-12/IL-18 (50/100 ng/ml) (n=10 donors) in the presence or absence of SGG (10 µg/ml).

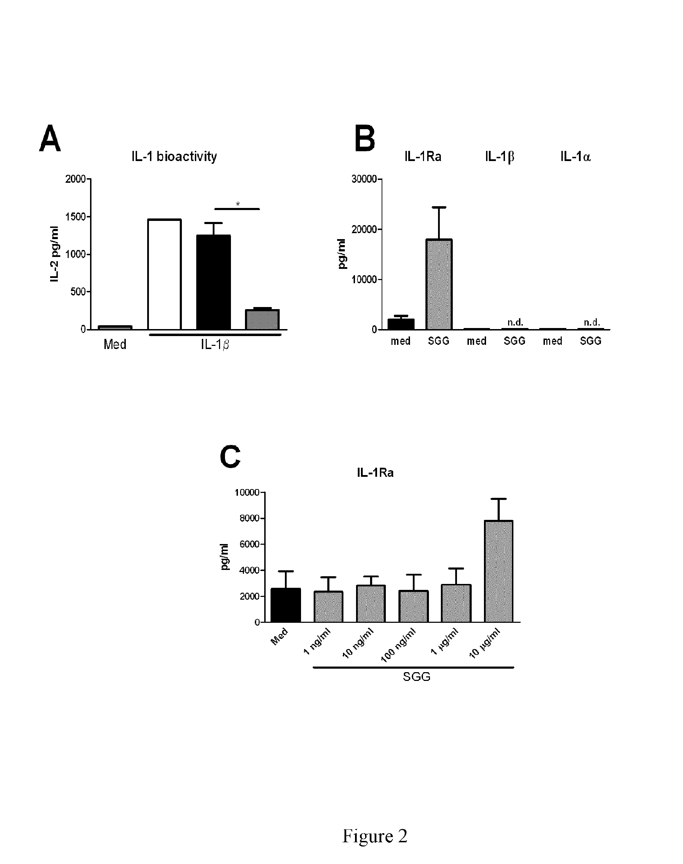

Figure 2: SGG induces interleukin 1 receptor antagonist. (a) IL-1 bioactivity measured as IL-2 production by NOB-1 cells stimulated with 50 ng/ml IL-1β in the presence of medium of PBMCs of SGG conditioned medium (n=6 donors). (b) IL-1 RA, IL-1β and IL-1α concentrations in culture supernatants of human PBMCs stimulated with for 24hours with 10 µg/ml SGG. (c) Dose response of SGG ranging from 1ng/ml to 10µg/ml.

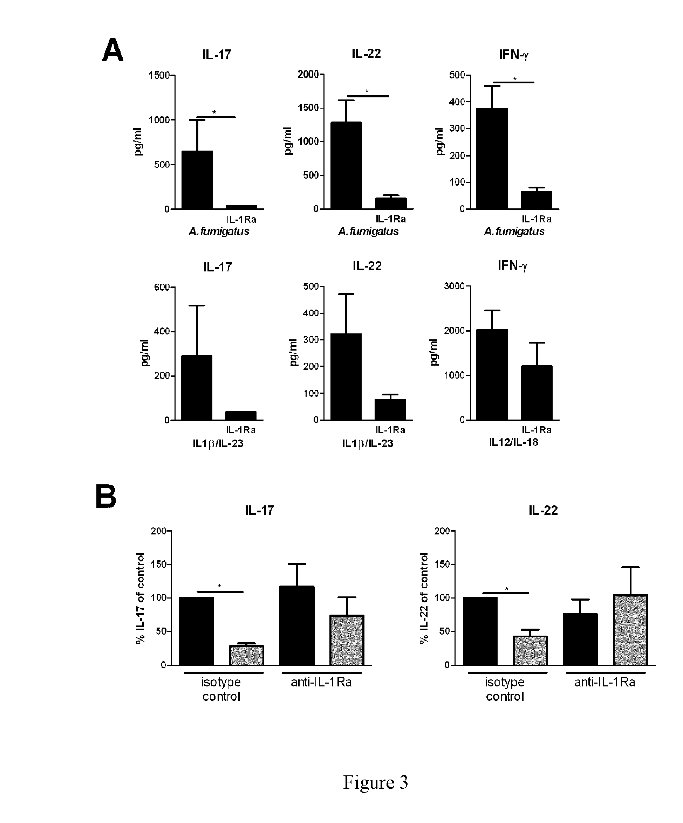

Figure 3: Suppression of IL-17 and IL-22 by soluble galactosaminogalactan is dependent on IL-1 RA. (a) IL-17, IL-22 and IFNγ concentrations in culture supernatants of PBMCs stimulated for 7 days with heat inactivated *A.fumigatus* conidia 1x107/ml, IL-1β/IL-23 (50/100 ng/ml) or IL-12/IL-18 (50/100 ng/ml) in the presence or absence of recombinant human IL-1 RA (10 ng/ml). (b) Inhibition of IL-1β/IL-23 (50/100 ng/ml) induced IL-17 and IL-22 by SGG (10 µg/ml) in human PBMCs in the presence of isotype control (10 µg/ml) or anti-IL-1 RA (10 µg/ml). The IL-17 and IL-22 production by IL-1β/IL-23 in absence of SGG was set at 100%.

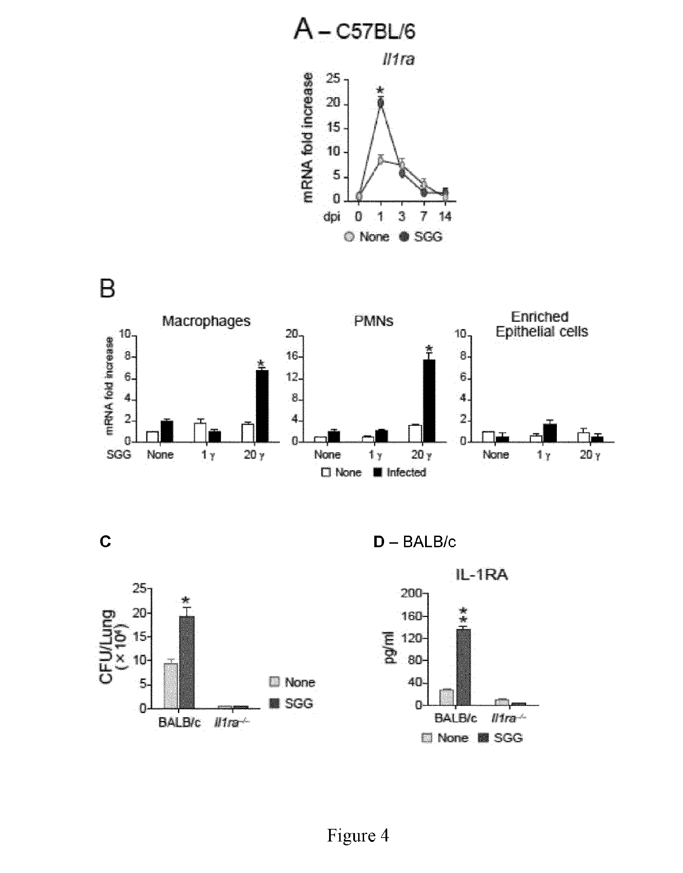

Figure 4: Soluble galactosaminogalactan induces IL-1 RA in vivo and IL-1 RA increases susceptibility to aspergillosis. (a) SGG induces IL-1ra mRNA expression in vivo in C57BL/6 mice during pulmonary aspergillosis and in vitro (b) on purified cells from the lungs of naive mice prestimulated with Aspergillus conidia for 1 h and exposed to different SGG concentrations for 18h. (c) CFUs of SGG (250 mg/Kg intranasally the day of infection and on days 1 to 3 post infection) on WT and IL-IraKO mice. (d) Levels of IL-1ra induced in BALB/c mice and IL-1raKO mice by SGG (day 4 after the infection and SGG treatment).

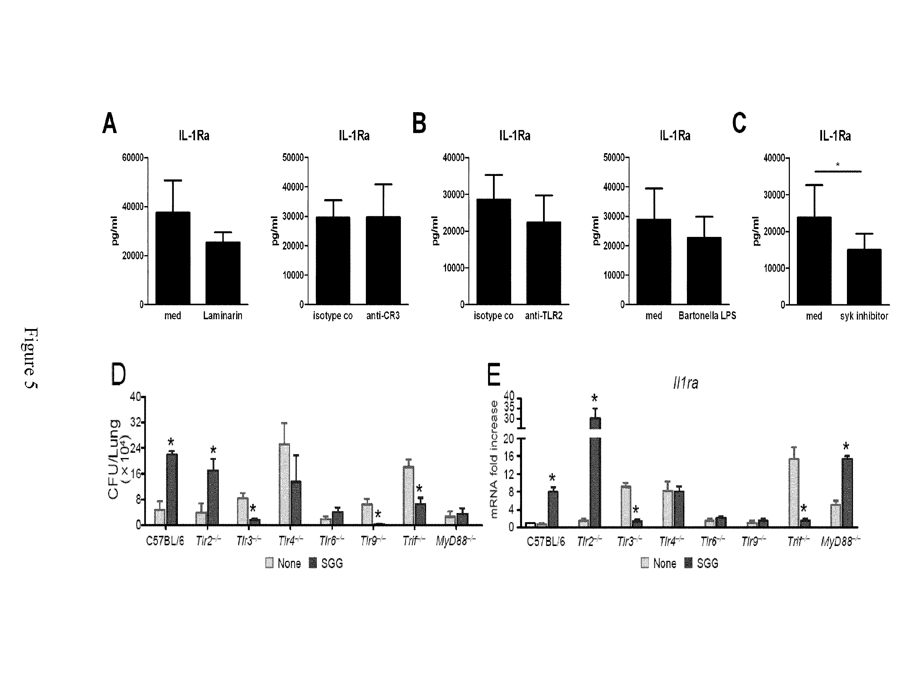

Figure 5: Induction of IL-1 RA by SGG is dependent on Syk and TLR3/TRIF. IL-1 RA concentrations in culture supernatants of human PBMCs stimulated with for 24hours with 10 µg/ml SGG in the presence or absence of (a) medium, Laminarin (50 ng/ml), anti-CR3 (10 µg/ml), (b) anti-TLR2 (10 µg/ml), bartonella LPS (20 ng/ml), or (c) syk inhibitor (10nM). (d) Lung CFU and (e) IL-1ra mRNA expression in TLR-deficient, MyD88-deficient, and TRIF-deficient mice infected with Aspergillus conidia and treated with SGG (4 days after the infection).

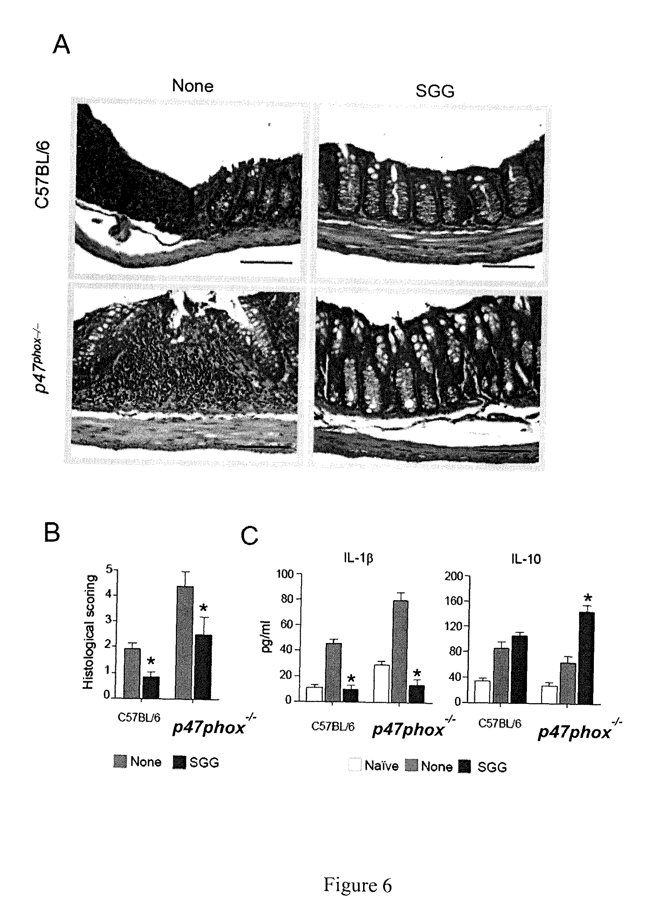

Figure 6: Measure of the inflammatory colonic pathology (Figure 6A), damage score (Figure 6B) and inflammatory IL-1b production and anti-inflammatory IL-10 production (Figure 6C) in DSS-induced colitis in WT and p47phox-/- mice treated with SGG or control (no SGG).

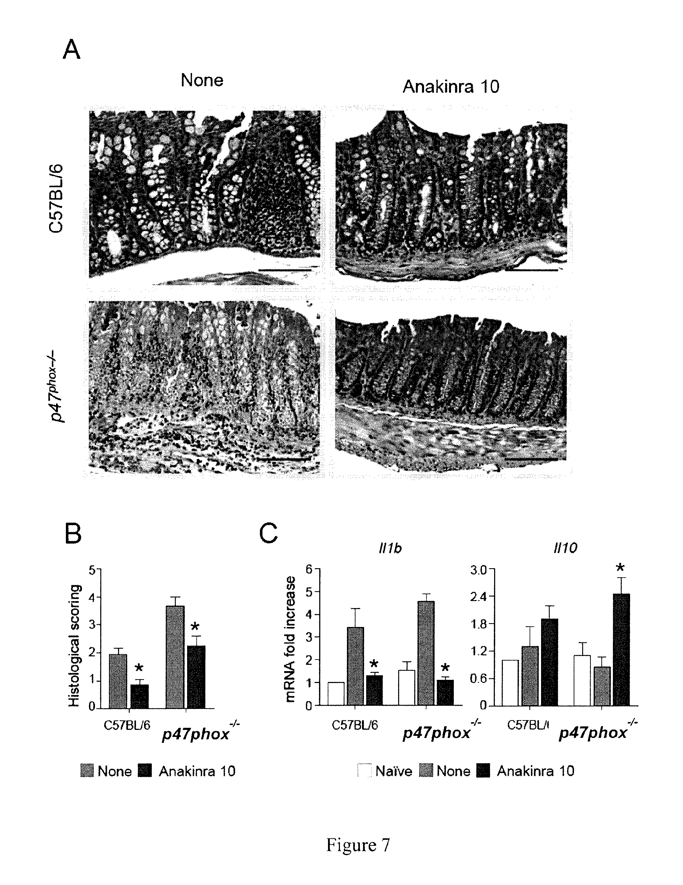

Figure 7: Measure of the inflammatory colonic pathology (Figure 7A), damage score (Figure 7B) and inflammatory IL-1b production and anti-inflammatory IL-10 production (Figure 7C) in DSS-induced colitis in WT and p47phox-/- mice treated with Anakinra or control (no Anakinra).

### DESCRIPTION

A first object of the invention is galactosaminogalactan comprising α1-4 linked galactose and α1-4 linked N-acetylgalactosamine for use in the treatment of at least one inflammatory disease.

Thus, the invention also has for object the use of galactosaminogalactan comprising α1-4 linked galactose and α1-4 linked N-acetylgalactosamine for the manufacture of a medicament intended for in the treatment of at least one inflammatory disease.

Thus, the invention also has for object a method for the treatment of at least one inflammatory disease in a human subject in need thereof, comprising administering an effective amount of galactosaminogalactan comprising α1-4 linked galactose and α1-4 linked N-acetylgalactosamine.

Another objet of the invention is a pharmaceutical composition comprising a galactosaminogalactan comprising α1-4 linked galactose and α1-4 linked N-acetylgalactosamine as defined in any of the above-cited claims, and optionally a pharmaceutically acceptable carrier.

Another object of the invention is a pharmaceutical composition according to the invention, for use in the treatment of at least one inflammatory disease.

Thus, the invention also has for object the use of a pharmaceutical composition according to the invention for the manufacture of a medicament intended for in the treatment of at least one inflammatory disease.

Thus, the invention also has for object a method for the treatment of at least one inflammatory disease in a human subject in need thereof, comprising administering an effective amount of a pharmaceutical composition according to the invention.

The inventors have further demonstrated that inhibitors of Il-1 RA can efficiently prevent and/or treat human fungal diseases.

Another object of the invention is an inhibitor of IL-1 RA for use in the treatment of human fungal diseases.

The object of the invention is therefore also the use of an inhibitor of IL-1 RA for the manufacture of a medicament intended for the treatment of human fungal diseases.

Another object of the invention is thus a method for the treatment of human fungal diseases in a human subject in need thereof, comprising administering an effective amount of an inhibitor of IL-1 RA

Another object of the invention is a pharmaceutical composition comprising an inhibitor of IL-1 RA, and optionally a pharmaceutically acceptable carrier.

Another object of the invention is thus a pharmaceutical composition an comprising an inhibitor of IL-1 RA according to the invention for use in the treatment of human fungal diseases.

The object of the invention is therefore also the use of a pharmaceutical composition comprising an inhibitor of IL-1 RA according to the invention for the manufacture of a medicament intended for the treatment of human fungal diseases.

Another object of the invention is thus a method for the treatment of human fungal diseases in a human subject in need thereof, comprising administering an effective amount of a pharmaceutical composition comprising an inhibitor of IL-1 RA according to the invention.

### DETAILED DESCRIPTION

The inventors have found that a polysaccharide present in the cell wall of filamentous fungi can inhibit IL-1 receptor signaling. This polysaccharide, a galactosaminogalactan comprising α1-4 linked galactose and α1-4 linked N-acetylgalactosamine, induces the expression of IL-1 RA in vivo and in vitro, and therefore acts as a prodrug for IL-1 RA. The galactosaminogalactan comprising α1-4 linked galactose and α1-4 linked N-acetylgalactosamine is a potent inhibitor of IL-1 related inflammation, and can thus efficiently be used as a treatment for inflammatory diseases. Results obtained *in vivo,* in an animal model for inflammatory bowel disease, show that galactosaminogalactan comprising α1-4 linked galactose and α1-4 linked N-acetylgalactosamine is at least as efficient, if not more efficient that commercialized recombinant IL-1 RA.

A first object of the invention is thus a galactosaminogalactan comprising α1-4 linked galactose and α1-4 linked N-acetylgalactosamine for use in the treatment of at least one inflammatory disease.

Thus, the invention also has for object the use of a galactosaminogalactan comprising α1-4 linked galactose and α1-4 linked N-acetylgalactosamine for the manufacture of a medicament intended for in the treatment of at least one inflammatory disease.

Thus, the invention also has for object a method for the treatment of at least one inflammatory disease in a human subject in need thereof, comprising administering an effective amount of a galactosaminogalactan comprising α1-4 linked galactose and α1-4 linked N-acetylgalactosamine.

By "galactosaminogalactan", it is herein referred to a polysaccharide polymer composed of galactose and N-acetylgalactosamine residues. A "polymer" according to the invention is a compound, preferably a polysaccharide, consisting of repeating structural units. The galactosaminogalactan of the invention is thus a polysaccharide composed of galactose and N-acetylgalactosamine residues and consisting of repeating units. Preferably, the said N-acetylgalactosamine and galactose residues are linked to the rest of the polymer through covalent bonds. More preferably, the said N-acetylgalactosamine and galactose residues are linked to the rest of the polymer through α1-4 covalent bonds. The skilled person will easily realize that, in this case, the galactosaminogalactan of the invention is not branched, but linear. Hence, most preferably, the galactosaminogalactan comprising α1-4 linked galactose and α1-4 linked N-acetylgalactosamine of the invention is a linear polysaccharide chain composed of galactose and N-acetylgalactosamine residues linked through α1-4 covalent bonds.

The galactosaminogalactan of the invention can comprise a regular alternation of the galactose and N-acetylgalactosamine residues over the whole length of the polymer. A "regular alternation" in the context of the invention means that the repetition of a specific unit can be identified throughout the whole molecule. Alternatively, the galactose and N-acetylgalactosamine residues are randomly distributed throughout the whole polymer. By "randomly distributed", it is herein referred to a random distribution of galactose and N-acetylgalactosamine residues on the overall length of each polymer. This definition does not exclude the existence of local patterns, i.e. that galactose and N-acetylgalactosamine be distributed according to specific patterns in some parts of the polymer. Preferably, the galactose and N-acetylgalactosamine residues are randomly distributed along the polysaccharide chain of said galactosaminogalactan.

In a preferred embodiment, said galactosaminogalactan has an average ratio of galactose/N-acetylgalactosamine of between 1/99 and 99/1. More preferably, said galactosaminogalactan has an average ratio of galactose/N-acetylgalactosamine of between 40/60 and 60/40. Even more preferably, said galactosaminogalactan polymer has an average ratio of galactose/N-acetylgalactosamine of around 50/50. By "ratio of galactose/N-acetylgalactosamine", it is herein referred to the molar ratio of galactose/N-acetylgalactosamine. The average ratio of galactose/N-acetylgalactosamine can easily be determined by gas liquid chromatography analysis, NMR spectroscopy, Matrix-assisted desorption ionisation/Time of flight (MALDI-TOF) mass spectrometry, or any other technique known in the art for identifying and quantifying the constituents of a polysaccharide chain,.

According to a preferred embodiment, the molecular weight of the galactosaminogalactan of the invention is comprised between 10 and 1000 kDa. More preferably, said galactosaminogalactan has an average molecular weight of 100 kDa. The molecular weight of the galactosaminogalactan polymer can easily be estimated by any technique known to the person skilled in the art, such as e.g. size-exclusion chromatography (also called gel permeation chromatography or gel filtration chromatography). By the term "average molecular weight" of the galactosaminogalactan, it is herein referred to the average of the molecular weights of a population of galactosaminogalactan molecules.

In a particular embodiment of the invention, said galactosaminogalactan is soluble in urea. Preferably, said galactosaminogalactan is soluble in 8M urea.

The galactosaminogalactan of the invention is obtained by chemical synthesis. The said galactosaminogalactan can be synthetized by any technique known to the person of skills in the art. Alternatively, the said galactosaminogalactan is a naturally occurring polymer, e.g. a polymer obtained from biological sources. galactosaminogalactans have been described in various biological organisms. In particular, they are found in the cell wall of filamentous fungi, including *Penicillium frequentans* (Guerrero et al., Microbiologia, 4(1):39-46, 1988), *Aspergillus parasiticus* (Ruperez et al., Trans Br Mycol Soc, 77(3): 621-625, 1981), *Aspergillus niger* (Bardalaye & Nordin, J Bacteriol, 125(2): 655-669 , 1976), *Aspergillus fumigatus* (Fontaine et al., PLoS Pathog., 7(11):e1002372, 2011; Sheppard, Curr Opin Microbiol, 14(4): 375-379, 2011). In particular, it was previously shown that *Aspergillus fumigatus* comprises a cell wall component that is shed into the environment during *Aspergillus* growth. This cell wall component is a linear heterogeneous galactosaminogalactan composed of α1-4 linked galactose and α1-4 linked N-acetylgalactosamine residues (Fontaine et al., PLoS Pathog., 7(11):e1002372, 2011). The galactosaminogalactan of the invention can hence be isolated from a culture supernatant of *Aspergillus fumigatus.*

In a particular embodiment of the invention, said galactosaminogalactan is obtained from *Aspergillus fumigatus.*

By "*Aspergillus fumigatus",* it is herein referred to a filamentous fungus of the genus *Aspergillus,* which genomic sequence was published in Nierman et al. (Nature 438, 1151-1156, 2005). *Aspergillus fumigatus* is a ubiquitous fungus that usually develops on decaying plant material and in soil. Therefore, the skilled person can use any isolate obtained from such samples. Any known strain or isolate of *Aspergillus fumigatus* can be used in the context of the invention. The skilled person can also use for example the reference strain CBS 144-89.

Preferably, the galactosaminogalactan of the invention is obtained from the mycelium. By "mycelium", it is herein referred to the vegetative part of a filamentous fungus, preferably *Aspergillus fumigatus,* consisting of a mass of long, branching filamentous structures called hyphae. Various methods are known for obtaining the mycelium of *Aspergillus fumigatus; Aspergillus fumigatus* mycelium can thus easily be obtained from an *Aspergillus fumigatus* culture for example by filtration, preferably under vacuum.

In a particular embodiment of the invention, said galactosaminogalactan is obtained from *Aspergillus fumigatus* by a process comprising the culture of *Aspergillus fumigatus,* advantageously of *Aspergillus fumigatus* mycelium.

The skilled person may use any technique known in the art in order to obtain the galactosaminogalactan of the invention from *Aspergillus fumigatus.* Advantageously, the inventors have found that soluble galactosaminogalactan can be extracted from the total fungal carbohydrates by a urea extraction step.

In a particular embodiment of the invention, said galactosaminogalactan is thus obtained from *Aspergillus fumigatus* by a process comprising an extraction step by urea.

By "extraction by urea" or "urea extraction", it is herein referred to a step of treating a product with a urea solution in view of partial or total solubilisation of said product, and recovery of the soluble fraction. By "soluble fraction obtained from extraction by urea" or "urea-soluble fraction", it is herein referred to the soluble fraction that can be recovered by the skilled person after urea extraction. Preferably, the extraction step in urea is performed in 8 M urea.

Thus, according to the present embodiment, said galactosaminogalactan is obtained from *Aspergillus fumigatus* by a process comprising a step of treatment with urea and recovery of the urea-soluble fraction. Preferably, said galactosaminogalactan is obtained from a mycelium of *Aspergillus fumigatus* by a process comprising a step of treatment with urea and recovery of the urea-soluble fraction.

In a preferred embodiment of the invention, said galactosaminogalactan is obtained from *Aspergillus fumigatus* by a process comprising a step of growing *Aspergillus fumigatus* and a step of treating said *Aspergillus fumigatus* culture with urea and recovering the urea-soluble fraction. *Aspergillus fumigatus* has been a model organism for years (Latgé, Clin Microbiol Rev, 12(2): 310-350, 1999). Methods for growing this fungus are thus well known to the skilled person and do not require to be thoroughly explained herein. For example, methods for isolation and culture of *Aspergillus fumigatus* are described in Nieminen et al. (Appl Environ Microbiol.; 68(10):4871-5; 2002). An example of such a method is detailed in the experimental examples section. More preferably, *Aspergillus fumigatus* is grown for at least 30 hours. Still more preferably, *Aspergillus fumigatus* is grown for at least 50 hour.

Preferably, the culture of *Aspergillus fumigatus* is filtered prior to the extraction step by urea. More preferably, the culture of *Aspergillus fumigatus* is filtered under vacuum prior to the extraction step by urea.

In order to increase the yield and purity of the galactosaminogalactan, tit is advantageous to deplete the culture of *Aspergillus fumigatus* from glycoproteins and galactomannans by incubation in a NaCl solution, prior to the extraction step in urea. By "incubation in NaCl", it is herein referred to incubation in an NaCl solution. The insoluble fraction, or NaCl-insoluble fraction, corresponds to all the material which cannot be solubilized in the NaCl solution. The inventors have found that said NaCl-insoluble fraction comprises the galactosaminogalactan of the invention, while most of the glycoproteins and galactomannans remain soluble in the NaCl solution.

Therefore, in a preferred embodiment, the process of preparation of said galactosaminogalactan of the invention comprises a step of incubation in NaCl, advantageously prior to the extraction step in urea. According to this embodiment, the said galactosaminogalactan is obtained from *Aspergillus fumigatus* by a process comprising a step of growing *Aspergillus fumigatus,* a step of incubating said *Aspergillus fumigatus* in NaCl, and a step of treating the NaCl-insoluble fraction of the previous step with urea and recovering the urea-soluble fraction.

Preferably, the NaCl solution of the invention has a concentration comprised between 100 and 200 mM. More preferably, the concentration of the NaCl solution is comprised between 120 and 170 mM. Even more preferably, the concentration of the NaCl solution is of 150 mM. Preferably, the culture of *Aspergillus fumigatus* is filtered prior to the incubation in NaCl. More preferably, the culture of *Aspergillus fumigatus* is filtered under vacuum prior to the incubation in NaCl.

In order to further increase the yield and purity of the galactosaminogalactan, the skilled person can additionally precipitate the culture of *Aspergillus fumigatus* in an alcohol solution, advantageously prior to the extraction step in urea.

Preferably, the process of the invention comprises a step of alcohol precipitation, advantageously prior to the extraction step in urea. More preferably, the step of alcohol precipitation is performed prior to incubation in a NaCl solution.

By "alcohol precipitation", it is herein referred to the incubation of a compound, preferably a polysaccharide, in an alcohol solution and recovery of the precipitate. The "precipitate obtained from alcohol precipitation" corresponds to the insoluble fraction that can be recovered by the skilled person after alcohol precipitation. By "alcohol solution", it is herein referred to any aqueous solution comprising an alcohol, i.e. an organic compound comprising a hydroxyl functional group (-OH) bound to a carbon atom. Preferably, the alcohol compound is chosen from ethanol, methanol, isopropyl alcohol and butyl alcohol. More preferably, the alcohol compound is ethanol. Preferably, the alcohol solution comprises more than 50, 60, 70, 80, 90 % of alcohol by weight compared to the total weight of the solution. More preferably, the alcohol solution comprises between 60 and 80% of alcohol by weight compared to the total weight of the solution. Even more preferably, the alcohol solution comprises 70% of alcohol by weight compared to the total weight of the solution.

Preferably, the culture of *Aspergillus fumigatus* is filtered prior to the alcohol precipitation. More preferably, the culture of *Aspergillus fumigatus* is filtered under vacuum prior to the alcohol precipitation.

In a particular embodiment of the invention, said galactosaminogalactan is obtained from *Aspergillus fumigatus* by a process comprising the following steps:
a) Culture of *Aspergillus fumigatus*;
b) Alcoholic precipitation of the culture obtained from step a);
c) Incubation of the precipitate obtained from step b) in NaCl,
d) Extraction of the insoluble fraction obtained from step c) by urea.

As explained above, it has been surprisingly found that the galactosaminogalactan of the invention is capable of inhibiting IL-1-mediated inflammation. Additionally, the inventors have found that the galactosaminogalactan of the invention is capable of inducing the expression of IL-1 RA both *in vitro* and *in vivo.*

The interleukin-1 receptor antagonist (IL-1RA) (NCBI ref.: NP_000568.1) is a protein that in humans is encoded by the IL1RN gene (NCBI ref.: NG_021240.1). The IL-1 RA protein binds non-productively to the cell surface interleukin-1 receptor (IL-1R), i.e. without activating the IL-1R receptor, while it inhibits the binding of IL1-alpha and IL1-beta. IL-1 RA is used in the treatment of inflammatory diseases, among which rheumatoid arthritis, an autoimmune disease in which IL-1 plays a key role. It is commercially produced as anakinra, which is a human recombinant form of IL-1RA.

In a particular embodiment of the invention, said galactosaminogalactan induces the expression of IL-1 RA. The induction of IL-1 RA may be determined by measuring the concentration of IL-1 RA at the transcript level, and/or at the protein level. Without being bound by theory, it is thought that the increase in the IL-1 RA protein levels resulting from the increased gene expression, leads to an inhibition of the IL-1R receptor, thus blocking the activation of the IL-1 mediated inflammation pathway.

According to this embodiment, administration of the galactosaminogalactan of the invention to a subject results in an induction of the expression of IL-1 RA. This induction can be detected by taking a biological sample from the said subject, measuring the level of expression of IL-1 RA, and comparing said level to a control. Said control is advantageously obtained from a subject to whom said galactosaminogalactan has not been administered. This control can be obtained any subject from the general population who has not received the galactosaminogalactan administration. Preferably, this control is obtained from the subject who is treated, prior to the administration of the said galactosaminogalactan.

A "biological sample" may be any sample that may be taken from a subject. Such a sample must allow for the determination of the expression levels of the IL-1 RA protein or transcript. More specifically, a biological sample is a subset of biological tissues from an organism, its cells or component parts (e.g., body fluids, including but not limited to, blood, mucus, lymphatic fluid, synovial fluid, cerebrospinal fluid, saliva, amniotic fluid, amniotic cord blood, urine, vaginal fluid and semen). A biological sample further refers to a homogenate, lysate or extract prepared from a subset of biological tissues from an organism, its cells or component parts, or a fraction or portion thereof, including but not limited to, for example, blood, blood cells, plasma, serum, spinal fluid, lymph fluid, the external sections of the skin, respiratory, intestinal, and genitourinary tracts, tears, saliva, milk, organs. Most often, the sample has been removed from a subject. Preferably, the biological sample is blood.

The method for detecting IL-1 RA expression according to the invention may thus comprise a preliminary step, between the taking of the sample from the patient and the measuring of the concentration of IL-1 RA at the transcript level, and/or at the protein level, said step corresponding to the transformation of the biological sample into a mRNA (or corresponding cDNA) sample or into a protein sample, which is then ready to use for in vitro measuring of genes expression level or protein level. Preparation or extraction of mRNA (as well as retrotranscription into cDNA) or proteins from a tissue sample is only routine procedure well known to those skilled in the art.

Once a ready-to-use mRNA (or corresponding cDNA) or protein sample is available, the measure of IL-1 RA gene expression levels may be performed, depending on the type of transformation and the available ready-to-use sample, either at the mRNA (i.e. based on the mRNA content of the sample) or at the protein level (i.e. based on the protein content of the sample).

By "measuring the concentration of IL-1 RA at the transcript level" it is herein referred to measuring the quantity and/or the concentration of the transcript IL-1 RA (NCBI ref.: NM_000577.4), which is the transcript of the IL1RN gene (NCBI ref.: NG_021240.1). Measuring the concentration of IL-1 RA at the transcript level can be done with any technique used to measure and quantify nucleic acids.

When expression levels are thus measured at the mRNA level, it may be notably performed using well known technologies such as quantitative PCR or nucleic acid microarray technologies (including cDNA and oligonucleotide microarrays). These technologies are now used routinely by those skilled in the art and thus do not need to be detailed here. Alternatively, any known or future technology permitting to assess genes expression levels based on mRNA contents may be used. For instance, tissue microarrays coupled to fluorescent in situ hybridization may be used. Tissue microarrays (also known as TMAs) consist of paraffin blocks in which up to 1000 separate tissue cores are assembled in array fashion to allow multiplex histological analysis. In the tissue microarray technique, a hollow needle is used to remove tissue cores as small as 0.6 mm in diameter from regions of interest in paraffin-embedded tissues such as clinical biopsies or tumor samples. These tissue cores are then inserted in a recipient paraffin block in a precisely spaced, array pattern. Sections from this block are cut using a microtome, mounted on a microscope slide and then analyzed by any method of standard histological analysis. Each microarray block can be cut into 100 - 500 sections, which can be subjected to independent tests. Tests commonly employed in tissue microarray include immunohistochemistry, and fluorescent in situ hybridization. For analysis at the mRNA level, tissue microarray technology may be coupled to fluorescent in situ hybridization.

In a preferred embodiment, the concentration of IL-1 RA at the transcript level is measured using quantitative PCR. Quantitative, or real-time, PCR is a well-known and easily available technology for those skilled in the art and does not need a precise description.

By "measuring the concentration of IL-1 RA at the protein level" it is herein referred to measuring the quantity and/or the concentration of the protein IL-1 RA (NCBI ref.: NP_000568.1).

When IL-1 RA expression levels are measured at the protein level, it may be notably performed using specific antibodies, in particular using well known technologies such as cell membrane staining using biotinylation or other equivalent techniques followed by immunoprecipitation with specific antibodies, western blot, ELISA or ELISPOT, antibodies microarrays, or tissue microarrays coupled to immunohistochemistry. Other suitable techniques include FRET or BRET, single cell microscopic or histochemistery methods using single or multiple excitation wavelength and applying any of the adapted optical methods, such as electrochemical methods (voltametry and amperometry techniques), atomic force microscopy, and radio frequency methods, e.g. multipolar resonance spectroscopy, confocal and non-confocal, detection of fluorescence, luminescence, chemiluminescence, absorbance, reflectance, transmittance, and birefringence or refractive index (e.g., surface plasmon resonance, ellipsometry, a resonant mirror method, a grating coupler waveguide method or interferometry), cell ELISA, flow cytometry, radioisotopic, magnetic resonance imaging, analysis by mass spectrometry (MS), tandem mass spectrometry (MS-MS), MS 3; matrix-assisted laser desorption/ionization time-of-flight (MALDI-TOF) mass spectrometry; polyacrylamide gel electrophoresis (SDS-PAGE); HPLC-Mass Spectroscopy; Liquid Chromatography/Mass Spectrometry/Mass Spectrometry (LC-MS/MS)). All these techniques are well known in the art and need not be further detailed here.

An "antibody specific for IL-1 RA" is an immunoglobulin molecule or a derivative thereof, which is capable of binding selectively and reversibly to IL-1 RA. By "selectively binds" it is herein referred to the ability of antibodies to preferentially bind to IL-1 RA, in comparison with other antigens. Generally, the interaction of an antibody with an antigen, such as IL-1 RA, can be characterized in terms of a binding affinity, which is commonly expressed by the affinity constant. The affinity constant (also known as the association constant), Ka, is a numerical constant used to describe the binding affinity of two molecules at equilibrium. Preferably, an antibody will be said to be specific for IL-1 RA when the binding affinity of said antibody for said IL-1 RA will be superior to the binding affinity of the same antibody for unrelated antigens. The binding affinity can be measured using a variety of methods known to those skilled in the art including immunoblotting, immunoprecipitation analyzes, Radio-Immuno Assays, ELISAs, assays of antibodies by immunofluorescence microscopy, surface plasmon resonance (BiaCORE). Preferably, the affinity of an antibody specific for an antigen has an affinity constant of between about 10³ M⁻¹ and about 10¹² M⁻¹ for said antigen.

Techniques to produce polyclonal or monoclonal antibodies specific for IL-1 RA and/or for fragments thereof are well known to those skilled in the art and need not be described in detail herein. Polyclonal antibodies can be obtained by immunization, possibly by multiple immunizations, of an animal with said IL-1 RA, followed by recovery of serum from said animal and purification of the desired antibodies, in particular by affinity chromatography using the polypeptide used for the immunization. Monoclonal antibodies may be obtained by the hybridoma method described in Köhler et al. (Nature, 1975, 256 (5517): 495-497). Methods for preparation and use of antibodies, and the assays mentioned herein before are described in Harlow, E. and Lane, D. Antibodies: A Laboratory Manual, (1988), Cold Spring Harbor Laboratory Press. Alternatively, it is possible to use commercially available anti-IL-1 RA antibodies, such as for example the monoclonal mouse IgG2A Clone# 10309 (catalog number: MAB601) from R&D system, or the polyclonal goat IgG (catalog number: AF-201-NA) from R&D system.

Since galactosaminogalactan induces the expression of IL-1 RA, it is most useful in the treatment of diseases associated with so-called IL-1 diseases. Thus, in a particular embodiment of the invention, said inflammatory disease is an interleukin-1 mediated disease.

By "interleukin-1 mediated disease" it is herein referred to a spontaneous or experimental disease or medical condition that is associated with elevated levels of IL-1 in bodily fluids or tissue or if cells or tissues taken from the body produce elevated levels of IL-1 in culture. In many cases, such interleukin-1 mediated diseases are also recognized by the following additional two conditions: (1) pathological findings associated with the disease or medical condition can be mimicked experimentally in animals by the administration of IS and (2) the pathology induced in experimental animal models of the disease or medical condition can be inhibited or abolished by treatment with agents which inhibit the action of IL-1.In most interleukin-1 mediated diseases at least two of the three conditions are met, and in many interleukin-1 mediated diseases all three conditions are met.

A non-exclusive list of acute and chronic interleukin-1 (IL-1)-mediated inflammatory diseases includes but is not limited to the following: acute pancreatitis; ALS; Alzheimer's disease; cachexia/anorexia; asthma; atherosclerosis; chronic fatigue syndrome, fever; diabetes (e.g., insulin diabetes); glomerulonephritis; graft versus host rejection; hemohorragic shock; hyperalgesia, inflammatory bowel diseases; inflammatory conditions of a joint, including osteoarthritis, psoriatic arthritis and rheumatoid arthritis; ischemic injury, including cerebral ischemia (e.g., brain injury as a result of trauma, epilepsy, hemorrhage or stroke, each of which may lead to neurodegeneration); lung diseases (e.g.,ARDS); multiple myeloma; multiple sclerosis; myelogenous (e.g., AML and CML) and other leukemias; myopathies (e.g., muscle protein metabolism, esp. in sepsis); osteoporosis;Parkinson's disease; pain; pre-term labor; psoriasis; reperfusion injury; septic shock; side effects from radiation therapy, temporal mandibular joint disease, tumor metastasis; or an inflammatory condition resulting from strain, sprain, cartilage damage, trauma, orthopedic surgery, infection or other disease processes.

In a particular embodiment of the invention, said inflammatory disease is chosen from acute pancreatitis, ALS, Alzheimer's disease, cachexia/anorexia, asthma, atherosclerosis, chronic fatigue syndrome, fever, insulin diabetes, glomerulonephritis, graft versus host rejection, hemohorragic shock, hyperalgesia, inflammatory bowel diseases, inflammatory conditions of a joint, including osteoarthritis, psoriatic arthritis and rheumatoid arthritis, ischemic injury, including cerebral ischemia (e.g., brain injury as a result of trauma, epilepsy, hemorrhage or stroke, each of which may lead to neurodegeneration), lung diseases (e.g., ARDS), multiple myeloma, multiple sclerosis, myelogenous (e.g., AML and CML) and other leukemias, myopathies (e.g., muscle protein metabolism, esp. in sepsis), osteoporosis, Parkinson's disease, pain, pre-term labor, psoriasis, reperfusion injury, septic shock, side effects from radiation therapy, temporal mandibular joint disease, tumor metastasis, or an inflammatory condition resulting from strain, sprain, cartilage damage, trauma, orthopedic surgery, infection.

In a particular embodiment of the invention, said inflammatory disease is chosen from inflammatory bowel diseases.

In a particular embodiment of the invention, said inflammatory bowel disease is chosen from Crohn's disease, ulcerative colitis, diverticulitis, and infectious colitis.

In a particular embodiment of the invention, said inflammatory disease is chosen from rheumatoid arthritis, osteoarthritis, and other inflammatory conditions resulting from strain, sprain, trauma, infection, cartilage damage or orthopedic surgery.

In a particular embodiment of the invention, said inflammatory disease is chosen from inflammatory joint disease, multiple sclerosis, leukemia, ischemic injury, or reperfusion injury.

Further, the galactosaminogalactan of the invention may be administered via topical, enteral or parenteral administration including, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, intraventricular and intrasternal injection and infusion. The galactosaminogalactan of the invention may also be administered via oral administration or be administered through mucus membranes, that is, intranasally, sublingually, buccally or rectally for systemic delivery.

In a specific embodiment, for the treatment of inflammatory bowel diseases for example, it is preferred that the galactosaminogalactan of the invention be administered via oral administration or be administered through mucus membranes (mucosa).

In a specific embodiment, for the treatment of rheumatoid arthritis and osteoarthritis for example, it is preferred that the galactosaminogalactan of the invention be administered via intraarticular, subcutaneous, intramuscular or intravenous injection.

In a specific embodiment, for the treatment of brain injury as a result of trauma, epilepsy, hemorrhage or stroke, or for the treatment of graft-versus-host disease for example, it is preferred that the galactosaminogalactan of the invention be administered via intraventricular or intravenous injection.

Regardless of the manner of administration, the treatment of IL-1-mediated disease requires a dose or total dose regimen of the galactosaminogalactan of effective amounts, i.e., effective to prevent, reduce or alleviate symptoms of the disease, such as to counteract progressive mucosa destruction in the case of inflammatory bowel diseases.

The effective amount is calculated according to the general criteria in the medical field, such as the approximate body weight or surface area of the patient, the disease or condition to be treated or prevented, the severity of the disease, the route of administration, and the age, sex and medical condition of the patient. Further refinement of the calculations necessary to determine the appropriate dosage for treatment is routinely made by those skilled in the art.

The frequency of dosing depends on the disease and condition of the patient, as well as the pharmacokinetic parameters of the galactosaminogalactan used in the formulation, and the route of administration. The galactosaminogalactan may be administered once, or in cases of severe and prolonged disorders, administered daily in less frequent doses or administered with an initial bolus dose followed by a continuous dose or sustained delivery.

In order to facilitate their administration, it is advantageous to formulate the compounds of the invention into compositions.

In another aspect, the invention also provides a pharmaceutical composition comprising a galactosaminogalactan comprising α1-4 linked galactose and α1-4 linked N-acetylgalactosamine above, and optionally a pharmaceutically acceptable carrier.

A "pharmaceutically acceptable carrier" according to the invention is a compound, or a combination of compounds, contained in a pharmaceutical composition, that does not cause secondary reactions and that, for example, facilitates administration of the active compounds, increases its lifespan and/or effectiveness in the organism, increases its solubility in solution or improves its storage. Such pharmaceutical carriers are well-known and will be adapted by a person skilled in the art according to the nature and the administration route of the active compounds selected.

In an embodiment, the composition of the invention is preferably formulated for oral administration.

For oral administration, the composition of the invention is preferably formulated in the form of dosage units, such as ingestible tablets, buccal tablets, capsules, or in the form of elixirs, suspensions, syrups, wafers and the like.

The dosage unit can further contain the following: a binder such as gum tragacanth, acacia, corn starch or gelatin, excipients such as dicalcium phosphate, a disintegrating agent such as corn starch, alginic acid and the like, a lubricant such as magnesium stearate, a sweetening agent such as sucrose, lactose or saccharin, or a flavoring agent such as peppermint, oil of wintergreen or cherry or orange flavoring. Various other materials can be present as a coating or to otherwise modify the physical form of the dosage unit. For instance, tablets, pills or capsules can be coated with shellac, sugar or both.

In another embodiment, the composition of the invention is formulated for administration through mucosa.

Those of ordinary skill in the clinical arts will be familiar with formulations and vehicles for drug delivery into mucosa. Useful references in this regard are Chien (Novel Drug delivery system, Chapters 3 through 6 and 9, Marcel Dekker, 1992), and Pharmaceutical Dosage Forms and Drug Delivery Systems (ANSEL et al., 1994, WILLIAMS & WILKINS).

Administration through mucosa can be obtained by formulating the composition of the invention into sprays and the like (e.g., aerosol spray or pump spray and the like), solutions, or as gels. When formulated for administration through mucosa, the composition of the invention comprises a vehicle selected in the group comprising solutions, emulsions, microemulsions, oil-in-water emulsions, anhydrous lipids and oil-in-water emulsions, other types of emulsions.

In another embodiment, the composition of the invention is preferably formulated for parenteral administration.

Parenteral administration can be obtained by formulating the composition of the invention into injectable formulations. Injectable formulations frequently comprise mixtures of water, organic solvents and surfactants.

Preferably, the composition of the invention comprises an effective amount of galactosaminogalactan, as defined above.

Pharmaceutical compositions of the present invention may be administered with other therapeutics suitable for the indication being treated, such as for example a second anti-inflammatory drug. The galactosaminogalactan or pharmaceutical composition comprising thereof of the invention and any of one or more additional anti-inflammatory drugs may be administered separately or in combination. Information regarding anti-inflammatory drugs can be found in "The Merck Manual of Diagnosis and Therapy" (19th edition, Merck, Sharp & Dohme Research Laboratories, Merck & Co., Rahway, NJ (2011)).

Present treatment of IL-1 mediated diseases, as defined hereabove, including acute and chronic inflammation such as inflammatory conditions of a joint (e.g., rheumatoid arthritis) includes first line drugs for control of pain and inflammation, classified as non-steroidal anti-inflammatory drugs (NSAIDs), corticosteroids, slow acting anti-rheumatic drugs (SAARDs), or disease-modifying antiarthitric drugs (DMARDs).

In a particular embodiment of the invention, said pharmaceutical composition comprises at least a second anti-inflammatory compound chosen from non-steroidal anti-inflammatory drugs (NSAIDs), corticosteroids, slow acting anti-rheumatic drugs (SAARDs), or disease-modifying antiarthitric drugs (DMARDs).

Non-steroidal anti-inflammatory drugs (NSAIDs) include aspirin, ibuprofen, and naproxen. NSAIDs can be characterized into nine groups: (1) salicylic acid derivatives; (2) propionic acid derivatives; (3) acetic acid derivatives; (4) fenamic acid derivatives; (5) carboxylic acid derivatives; (6) butyric acid derivatives; (7) oxicams; (8) pyrazoles and (9) pyrazolones.

According to the invention, salicylic acid derivatives include prodrug esters and pharmaceutically acceptable salts thereof comprise: acetaminosalol, aloxiprin, aspirin, benorylate, bromosaligenin, calcium acetylsalicylate, choline magnesium trisalicylate diflusinal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide O-acetic acid, salsalate and sulfasalazine.

According to the invention, propionic acid derivatives include alminoprofen, benoxaprofen, bucloxic acid, carprofen, dexindoprofen, fenoprofen, flunoxaprofen, fluprofen, flurbiprofen, furcloprofen, ibuprofen, ibuprofen aluminum, ibuproxam, indoprofen, isoprofen, ketoprofen, loxoprofen, miroprofen, naproxen, oxaprozin, piketoprofen, pimeprofen, pirprofen, pranoprofen, protizinic acid, pyridoxiprofen, suprofen, tiaprofenic acid and tioxaprofen.

According to the invention, acetic acid derivatives include acemetacin, alclofenac, amfenac, bufexamac, cinmetacin, clopirac, delmetacin, diclofenac sodium, etodolac, felbinac, fenclofenac, fenclorac, fenclozic acid, fentiazac, furofenac, glucametacin, ibufenac, indomethacin, isofezolac, isoxepac, lonazolac, metiazinic acid, oxametacin, oxpinac, pimetacin, proglumetacin, sulindac, talmetacin, tiaramide, tiopinac, tolmetin, zidometacin and zomepirac.

According to the invention, fenamic acid derivatives include enfenamic acid, etofenamate, flufenamic acid, isonixin, meclofenamic acid, meclofenamate sodium, medofenamic acid, mefanamic acid, niflumic acid, talniflumate, terofenamate, tolfenamic acid and ufenamate.

According to the invention, carboxylic acid derivatives include clidanac, diflunisal, flufenisal, inoridine, ketorolac and tinoridine.

According to the invention, butyric acid derivatives include bumadizon, butibufen, fenbufen and xenbucin.

According to the invention, oxicams include droxicam, enolicam, isoxicam, piroxicam, sudoxicam, tenoxicam and 4-hydroxyl-1,2-benzothiazine 1,1-dioxide 4-(N-phenyl)-carboxamide.

According to the invention, pyrazoles include difenamizole and epirizole.

According to the invention, pyrazolones include apazone, azapropazone, benzpiperylon, feprazone, mofebutazone, morazone, oxyphenbutazone, phenylbutazone, pipebuzone, propylphenazone, ramifenazone, suxibuzone and thiazolinobutazone.

By corticosteroids, it is herein referred to analogues of steroid hormones naturally produced in the adrenal cortex of vertebrates, and that are chemically synthetized. According to the invention, corticosteroids include hydrocortisone and compounds which are derived from hydrocortisone, such as 21-acetoxypregnenolone, alclomerasone, algestone, amcinonide, beclomethasone, betamethasone, betamethasone valerate, budesonide, chloroprednisone, clobetasol, clobetasol propionate, clobetasone, clobetasone butyrate, clocortolone, cloprednol, corticosterone, cortisone, cortivazol, deflazacon, desonide, desoximerasone, dexamethasone, diflorasone, diflucortolone, difluprednate, enoxolone, fluazacort, flucloronide, flumethasone, flumethasone pivalate, flunisolide, flucinolone acetonide, fluocinonide, fluorocinolone acetonide, fluocortin butyl, fluocortolone, fluorocortolone hexanoate, diflucortolone valerate, fluorometholone, fluperolone acetate, fluprednidene acetate, fluprednisolone, flurandenolide, formocortal, halcinonide, halometasone, halopredone acetate, hydrocortamate, hydrocortisone, hydrocortisone acetate, hydrocortisone butyrate, hydrocortisone phosphate, hydrocortisone 21-sodium succinate, hydrocortisone tebutate, mazipredone, medrysone, meprednisone, methylprednicolone, mometasone furoate, paramethasone, prednicarbate, prednisolone, prednisolone 21-diedryaminoacetate, prednisolone sodium phosphate, prednisolone sodium succinate, prednisolone sodium 21-m-sulfobenzoate, prednisolone sodium 21-stearoglycolate, prednisolone tebutate, prednisolone 21-trimethylacetate, prednisone, prednival, prednylidene, prednylidene 21-diethylaminoacetate, tixocortol, triamcinolone, triamcinolone acetonide, triamcinolone benetonide and triamcinolone hexacetonide.

Slow acting anti-rheumatic drugs (SAARDs) have also been called Disease-modifying antiarthitric drugs (DMARDs) in the literature and include cyclosporine, azulfidine (sulfasalazine), methotrexate, imuran (azathioprine), cytoxan (cyclophosphamide), actemra, cimzia, enbrel, humira, kineret, orencia, remicade, rituxan, and simponi, Adalimumab, Azathioprine, Chloroquine, hydroxychloroquine, cyclosporin (Cyclosporin A), D-penicillamine, Etanercept, Golimumab, sodium aurothiomalate, auranofin, Infliximab, Leflunomide, Minocycline, Rituximab.

It is thus another object of the present invention to provide a pharmaceutical composition according to the invention, for use in the treatment of at least one inflammatory disease.

Thus, the invention also has for object the use of a pharmaceutical composition according to the invention for the manufacture of a medicament intended for in the treatment of at least one inflammatory disease.

Thus, the invention also has for object a method for the treatment of at least one inflammatory disease in a human subject in need thereof, comprising administering an effective amount of a pharmaceutical composition according to the invention.

In a particular embodiment of the invention, said inflammatory disease is an interleukin-1 mediated disease.

In a particular embodiment of the invention, said inflammatory disease is chosen from acute pancreatitis, ALS, Alzheimer's disease, cachexia/anorexia, asthma, atherosclerosis, chronic fatigue syndrome, fever, insulin diabetes, glomerulonephritis, graft versus host rejection, hemohorragic shock, hyperalgesia, inflammatory bowel diseases, inflammatory conditions of a joint, including osteoarthritis, psoriatic arthritis and rheumatoid arthritis, ischemic injury, including cerebral ischemia (e.g., brain injury as a result of trauma, epilepsy, hemorrhage or stroke, each of which may lead to neurodegeneration), lung diseases (e.g., ARDS), multiple myeloma, multiple sclerosis, myelogenous (e.g., AML and CML) and other leukemias, myopathies (e.g., muscle protein metabolism, esp. in sepsis), osteoporosis, Parkinson's disease, pain, pre-term labor, psoriasis, reperfusion injury, septic shock, side effects from radiation therapy, temporal mandibular joint disease, tumor metastasis, or an inflammatory condition resulting from strain, sprain, cartilage damage, trauma, orthopedic surgery, infection.

Filamentous fungi comprise opportunistic human fungal pathogens, such as e.g. *Aspergillus fumigatus,* that cause a wide range of diseases including allergic reactions and local or systemic infections. The galactosaminogalactan present in the filamentous fungi cell wall has immune-modulating properties through upregulation of IL-1 RA. The inventors have shown that the IL-1RA function is required for fungal infectivity. In particular, the induction or upregulation of IL-RA increases susceptibility to fungal infection. Mice devoid of the IL-1 RA gene are insensitive to infection by filamentous fungi.

Therefore, in another aspect, the present invention also relates to an inhibitor of IL-1 RA for use in the treatment of human fungal diseases.

In other words, the present invention also relates to the use of an inhibitor of IL-1 RA for the manufacture of a medicament intended for the treatment of human fungal diseases.

In still other words, the present invention relates to a method for the treatment of human fungal diseases in a human subject in need thereof, comprising administering an efficient amount of an inhibitor of IL-1 RA.

By "human fungal diseases" it is herein referred to diseases caused by fungi, and especially filamentous fungi. The skilled person may find the complete definition and list of human fungal diseases, in particular of invasive human fungal diseases in the "*Revised definitions of invasive fungal disease from the European Organization for Research and Treatment of Cancer*/*Invasive Fungal Infections Cooperative Group and the National Institute of Allergy and Infectious Diseases Mycoses Study Group (EORTC*/*MSG) Consensus Group."* (De Pauw et al.; Clin Infect Dis.; 46(12):1813-21. 2008). Human fungal diseases comprise fungus infection by any kind of fungus, Athlete's foot also known as *tinea pedis* (caused by fungi from the genus Trichophyton), and acute invasive pulmonary aspergillosis. Fungus infection may be diagnosed by histological analysis or culture of a specimen of tissue taken from a site of disease.

Over the past 25 years, *Aspergillus fumigatus* has become the most prevalent airborne fungal pathogen, causing severe and usually fatal invasive infections in immunocompromised hosts in developed countries. In a preferred embodiment of the invention, the human fungal disease of the invention is a disease caused by, or associated with, *Aspergillus fumigatus.* In an especially preferred embodiment of the invention, the human fungal disease is acute invasive pulmonary aspergillosis.

Inhibitors of IL-1 RA, as meant herein, encompass all compounds that are capable of inhibiting the expression and/or the activity of IL-1 RA.

By "inhibiting the expression of IL-1 RA", it is herein referred to the ability of a compound to reduce or annihilate the expression of the transcript IL-1 RA (NCBI ref.: NM_000577.4), and/or reduce or annihilate the expression of the protein IL-1 RA (NCBI ref.: NP_000568.1). The skilled person will clearly understand that the ability of a compound to reduce or annihilate the expression of IL-1 RA can be assessed by the above-disclosed methods for measuring IL-1 RA concentration at the transcript or at the protein level. Preferably, the concentrations of IL-1 RA measured when the inhibitor is used will be compared to a control devoid of said inhibitor. In that case, the level of the concentrations of IL-1 RA measured when the inhibitor is used is inferior or equal to those obtained with a control devoid of said inhibitor.

By "inhibiting the activity of IL-1 RA", it is herein referred to the ability of a compound to reduce or annihilate the binding of IL-1 RA to the IL-1 receptor. The binding of a ligand to a receptor can easily be measured through conventional techniques that are well known in the art, such as immunohistochemistry, ELISA, western blot analysis, surface plasmon resonance (for example with the BIAcore technology), dual polarisation interferometry, and Microscale Thermophoresis (MST), as well as assays used in high throughput screening (HTS) applications, such as scintillation proximity assay (SPA). In the context of the invention, these assays can for example be performed using soluble IL-1 receptor domains, stabilization of IL-1 receptor into a membrane-like environment or direct use of solubilized IL-1 receptor. For a thorough description of such assays, the skilled person may refer to Jong et al. (J Chromatogr B Analyt Technol Biomed Life Sci.; 2005; 829(1-2):1-25).

According to the invention, inhibitors of IL-1 RA comprise antibodies and polynucleotides, including antisense polynucleotides, interfering RNAs (iRNAs), and small interfering RNAs (siRNAs). According to the invention, interfering RNA are double stranded RNA molecules which are able to inhibit expression of targeted gene products. According to the invention, small interfering RNA are iRNA of 20-25 base pairs in length. iRNAs and their use in inhibiting the expression of targeted gene product are well known from the skilled person and do not need to be further explained herein. For a thorough description of iRNA, siRNA, and their use, the skilled person can refer to Cejka et al. (Clinical Science, 110, 47-58; 2006) or Tuschl et al. (Chembiochem; 2, 239- 245; 2001).

Antibodies specific for an antigen can be potent inhibitors, in that they impede normal functioning of the protein, at least because of steric effect. In a particular embodiment of the invention, said inhibitor is an antibody specific for IL-1 RA. Inhibitors of IL-1 RA include antibodies specific for IL-1 RA, such as for example the monoclonal mouse IgG2A Clone# 10309 (catalog number: MAB601) from R&D system, the polyclonal goat IgG (catalog number: AF-201-NA) from R&D system.

Techniques to produce polyclonal or monoclonal antibodies specific for a polypeptide and/or for said peptide fragments are disclosed above.

In a particular embodiment of the invention, said inhibitor is a polynucleotide.

By polynucleotide, it is herein referred to any nucleic acid sequence, that is to say any polyribonucleotide or polydeoxribonucleotide, which may be unmodified RNA or DNA or modified RNA or DNA. This includes, without limitation, single and double stranded DNA, DNA including single and double-stranded regions, single and double stranded RNA, and RNA including single and double stranded regions, hybrid molecules comprising DNA and RNA that may be single stranded or, more typically, double stranded or include single and double stranded regions. Also included are triple stranded regions comprising RNA or DNA or both RNA and DNA. Specifically included are mRNAs, cDNAs, and genomic DNAs, and any fragments, and modifications thereof.

More particularly, the agent is selected from the group consisting of: an antisense nucleic acid directed to the transcript IL-1 RA (NCBI ref.: NM_000577.4), a nucleic acid adapted to express such antisense, iRNA directed to the transcript IL-1 RA (NCBI ref.: NM_000577.4), and a nucleic acid adapted to express such iRNA.

In another particular embodiment, the present invention encompasses an isolated iRNA (e.g., siRNA) directed to the transcript IL-1 RA (NCBI ref.: NM_000577.4), or a nucleic acid adapted in use to express an iRNA directed to the transcript IL-1 RA (NCBI ref.: NM_000577.4). According to the invention, the isolated iRNA can comprise a sense strand and an antisense strand which form a duplex. Such antisense polynucleotides and iRNAs, in particular, siRNAs, can inhibit expression of the protein IL-1 RA (NCBI ref.: NP_000568.1).

Another object of the invention is a pharmaceutical composition comprising an inhibitor of IL-1 RA, and optionally a pharmaceutically acceptable carrier.

Pharmaceutically acceptable carriers have been defined above.

In a particular embodiment of the invention, said inhibitor is an antibody specific for IL-1 RA.

In an embodiment, the composition of the invention is preferably formulated for oral administration.

For oral administration, the composition of the invention is preferably formulated in the form of dosage units, such as ingestible tablets, buccal tablets, capsules, or in the form of elixirs, suspensions, syrups, wafers and the like.

Pharmaceutical compositions of the present invention may further comprise other therapeutic compounds suitable for the indication being treated. Human fungal disease can be treated with antifungal compounds.

In a particular embodiment of the invention, said pharmaceutical composition further comprises a second antifungal compound.

Antifungal compounds comprise polyene antifungals, imidazole antifungals, triazole antifungals, thiazole antifungals, allylamines and echinocandins.

According to the invention, polyene antifungals comprise amphotericin B, candicidin, filipin, hamycin, natamycin, nystatin, rimocidin.

According to the invention, imidazoles antifungals comprise bifonazole, butoconazole, clotrimazole, econazole, fenticonazole, isoconazole, ketoconazole, miconazole, omoconazole, oxiconazole, sertaconazole, sulconazole, tioconazole.

According to the invention, triazoles antifungals comprise albaconazole, fluconazole, isavuconazole, itraconazole, posaconazole, ravuconazole, terconazole, voriconazole.

According to the invention, thiazoles antifungals comprise abafungin.

According to the invention, allylamines comprise amorolfin, butenafine, naftifine, terbinafine.

According to the invention, echinocandins comprise anidulafungin, caspofungin, micafungin.

In particular, known therapeutic compounds suitable for the treatment of acute invasive pulmonary aspergillosis include steroids, voriconazole and liposomal amphotericin B.

In a particular embodiment of the invention, said pharmaceutical composition further comprises at least one compound chosen from steroids, voriconazole and liposomal amphotericin B.

The composition of the invention is particularly suited for the treatment of for use in the treatment of human fungal diseases.

Another object of the invention is thus a pharmaceutical composition comprising an inhibitor of IL-1 RA according to the invention for use in the treatment of human fungal diseases.

The object of the invention is therefore also the use of a pharmaceutical composition comprising an inhibitor of IL-1 RA according to the invention for the manufacture of a medicament intended for the treatment of human fungal diseases.

Another object of the invention is thus a method for the treatment of human fungal diseases in a human subject in need thereof, comprising administering an effective amount of a pharmaceutical composition comprising an inhibitor of IL-1 RA according to the invention.

In a particular embodiment of the invention, the human fungal disease is acute invasive pulmonary aspergillosis.

### EXPERIMENTAL RESULTS

### Methods

### Extraction of soluble galactosaminogalactan from A. fumigatus

The *A. fumigatus,* strain CBS 144-89 was grown in a 151 fermenter in modified Brian medium (2% asparagine, 5% glucose, 2.4 g/l NH4NO3, 10 g/l KH2PO4, 2 g/l MgSO4-7H2O, 26 mg/l ZnSO4-7H2O, 2.6 mg/l CuSO4-5H2O, 1.3 mg/l Co(NO3)2-6H2O, 65 mg/l CaCl2, pH 5.4) for 72 h at 25°C. The mycelium was removed by filtration under vacuum and the supernatant was precipitated with 2.5 vol. of ethanol overnight at 4°C. The pellet was collected by centrifugation (3000g, 10 min). The pellet was washed twice with 2.5 1 of 150 mM NaCl and then extracted with 8 M urea (2 h twice at room temperature under shaking). Ureasupernatants (SGG) were pooled and extensively dialyzed against water and freeze-dried. Urea-insoluble pellet (PGG) was washed with water and freeze-dried.

Soluble galactosaminogalactan (SGG) was used in a final concentration of 10 µg/ml. Before using SGG in stimulation experiments it was incubated with polymixin B to neutralize potential contamination of lipopolysaccharide.

### Stimuli and reagents

A clinical isolate of Aspergillus fumigatus V05-27, which has been characterized previously was used for stimulations (Netea et al., 2003). Conidia and hyphae were prepared and heat-inactivated (HI) as previously described (Chai et al., 2009a). A concentration of 1x107/ml was used in the experiments, unless otherwise indicated. C. albicans, strain ATCC MYA-3573 (UC820) (Gow et al., 2007; Lehrer and Cline, 1969). Candida were heat-killed (HK) at 98□C for 1 hour and used in a final concentration of 1x107/ml. Recombinant human IL-1β, IL-23, IL-12 and IL-18 were purchased from R&D Systems (Minneapolis, MN, USA) and were used in end concentrations of 100 ng/ml, 50 ng/ml, 10 ng/ml and 50 ng/ml respectively. Recombinant human (rh) IL-1Ra (Amgen, Inc., Thousand Oaks, CA, USA) was used to antagonize IL-1β signalling at a final concentration of 10 ng/ml. Anti-humanIL-1Ra (R&D) was used to block IL-1Ra in a final concentration of 10 µg/ml, and was compared to isotype control. Pattern recognition pathways were inhibited in PBMCs by pre-stimulation for 1 hour with a specific inhibitor. LPS derived from Bartonella quitana was used to block TLR4 (Abdollahi-Roodsaz et al., 2007). B. quitana LPS was extracted and purified as described previously (Hirschfeld et al., 1999). Mouse anti-humanTLR-2 (eBioscience, Halle-Zoersel, Belgium) and control mouse IgG1 (eBioscience) were used at a final concentration of 10 µg/mL. Anti-human integrin β2 (αCR3) and control goat IgG were purchased from R&D systems (Minneapolis, MN, USA) and used in a final concentration of 10 µg/mL. Laminarin was kindly provided by Professor David Williams of Tennessee University and was used in a final concentration of 50 ng/mL to inhibit dectin-1. Syk kinase inhibitor was purchased from Calbiochem (Merck, Darmstadt, Germany) and was used in a concentration of 50 nM to inhibit Syk signaling.

### PBMC isolation and stimulation

Venous blood from healthy volunteers was drawn into 10 ml EDTA tubes after written informed consent, after which PBMCs were isolated as previously described (van de Veerdonk et al., 2009). Briefly, blood was diluted in phosphate buffered saline (PBS) (1:1) and fractions were separated by Ficoll (Ficoll-Paque Plus, GE healthcare, Zeist, The Netherlands) density gradient centrifugation. Cells were washed twice with PBS and resuspended in RPMI-1640 culture medium supplemented with 10µg/ml gentamicin, 10mM L-glutamine and 10mM pyruvate (Gibco, Invitrogen, Breda, The Netherlands). The cells were counted using a particle counter (Beckmann Coulter, Woerden, The Netherlands) and the cell number was adjusted to 5x106/ml. PBMCs were plated in 96 well roundbottom plates (Corning, NY, USA) at a final concentration of 2,5x106/ml and in a total volume of 200 µl. Cells were pre-stimulated for 1 hour with medium or 10µg/ml SGG. Following prestimulation, the PBMCs were stimulated with culture medium, HI A. fumigatus conidia (1x107/ml), IL-1β/IL-23 (100 and 50ng/ml respectively) or IL-12/IL-18 (10 and 50 ng/ml respectively). These experiments were also performed in the presence of 10µg/ml anti-IL-1Ra antibody or isotype control. Plates were incubated at 37°C, 5% CO2 for 24 hours, 48 hours or 7 days. 7 day cultures were supplemented with 10% human serum. After incubation, culture supernatants were collected and stored at -20°C until cytokine measurements were performed.

### IL-1 bioassay

The murine cell line NOB-1 responds to both human or mouse IL-1 by production of IL-2, furthermore these cells are unresponsive to other cytokines like tumor necrosis factor (TNF), colony stimulating factors (CSFS), IL-3, IL-5, IL-6 and IFNγ (Gearing et al., 1987). NOB-1 cells were plated in 96 well flatbottom plates at a final density of 1x106 cells/ml and were stimulated for 24 hour using culture supernatants of PBMCs stimulated in presence or absence of SGG (as described above). After 24 hours of incubation at 37°C, 5% CO2 the culture supernatants of the NOB-1 cells were collected and IL-2 production by the NOB-1 cells was measured by ELISA (R&D systems).

### Cytokine measurement

Cytokines were measured using commercially available ELISAs (R&D Systems)(Sanquin, Amsterdam, The Netherlands) according to the protocols supplied by the manufacturer. IL-1β, TNF-α, IL-8 and IL-1Ra were measured in culture supernatants of 24 hour cultures, IFN-γ and IL-10 were measured in culture supernatants of 48h hour cultures, and IL-17 and IL-22 were measured in culture supernatants of 7 day cultures.

### Statistical analysis

The differences between the various stimulations were analyzed with the Wilcoxon signed rank test (p-value of <0.05 was considered statistically significant). All experiments were performed at least twice and data represent cumulative results of all experiments performed and are presented as mean +/- standard error of the mean (SEM) unless otherwise indicated. Data was analyzed using GraphPad Prism v 5.0.

### Results

### Soluble galactosaminogalactan modulates human T-helper cytokine responses

To investigate whether SGG can exert immunomodulatory effects in humans, we tested whether SGG induces the production of pro- and/or anti-inflammatory cytokines in human PBMCs. SGG did not induce the proinflammatory cytokines TNFα, IL-6, IL-8, IFNγ, and IL-17 (Figure 1A), neither did it induce the anti-inflammatory cytokine IL-10 (Figure 1A). To determine whether SGG modulates Aspergillus-induced innate monocyte-derived cytokines, we stimulated PBMCs for 24 hours with Aspergillus conidia in the presence or absence of SGG. The presence of SGG did not have a significant effect on the production of the innate cytokines TNFα and IL-6, or the anti-inflammatory cytokine IL-10 (Figure 1B). However, when we investigated the production of the characteristic T-helper cytokines IL-17, IL-22 and IFNγ induced by *A. fumigatus,* the IL-17 and IL-22 responses were significantly reduced in the presence of SGG (Figure 1C). To determine whether the effects of SGG are specific for Aspergillus-driven T-helper responses or that SGG has a general ability to modulate human T-helper responses, we investigated the effects of SGG on cytokine-driven T-helper responses. SGG significantly decreased the proinflammatory T-helper cytokine production induced by the cytokine combinations IL-1β/IL-23 and IL-12/IL-18 that induce IL-17/IL-22 and IFNγ respectively (Figure 1E). Thus, SGG can inhibit human proinflammatory T-helper cytokine production induced by Aspergillus and cytokine combinations.

### Soluble galactosaminogalactan induces IL-1 receptor antagonist.

Human T-helper cytokine responses such as IL-17 and IL-22 production are highly dependent on the IL 1 receptor pathway (Ben-Sasson et al., 2009; Guo et al., 2009). We wanted to investigate whether the observed modulation of these T-helper cytokines by SGG was due to an interaction of SGG with the IL 1 pathway. Therefore, we measured the capacity of SGG conditioned medium to reduce IL-1β bioactivity (figure 2A). Bioactivity of the IL 1 signalling pathway is dependent on IL 1 receptor agonists (IL-1α and IL-1β) and IL 1 receptor antagonists (Dinarello, 2011). One of the natural inhibitors of the IL-1 signalling is the interleukin-1 receptor antagonist (IL-1 RA), therefore we tested whether SGG can induce IL-1 RA. IL-1 RA concentrations in the supernatant of the cells stimulated with SGG were significantly increased compared to medium stimulated PBMCs (Figure 2B,C), suggesting that SGG has the capacity to modulate immune responses by inducing IL-1 RA. We next investigated the capacity of SGG to induce IL-1 receptor agonists, however SGG induced neither IL-1α nor IL-1β (Figure 2B). Thus, SGG induces only IL-1 antagonistic activity without contributing to IL 1 agonistic activity.

### Suppression of IL-17 and IL-22 by soluble galactosaminogalactan is dependent on IL-1 RA.

To demonstrate that IL-17, IL-22, and IFNγ production by human PBMCs is indeed dependent on the IL 1 receptor pathway and that IL-1 RA can inhibit the production of these T-helper cytokines, we performed experiments of Th1 and Th17 inducing stimuli in the presence or absence of IL-1 RA. Addition of IL-1 RA reduced IL-17, IL-22, and IFNγ induction by both Aspergillus conidia and by IL-1/IL-23 and IL-12/IL-18 cytokine combinations (Figure 3A). To determine whether the immunosuppressive effect of SGG was mediated through the induction of IL-1 RA, we stimulated PBMCs with IL-1β/IL-23 in the presence of SGG and blocked IL-1 RA with specific neutralizing antibodies. SGG reduced IL-17 and IL-22 levels significantly, which was not observed in the presence of neutralizing anti-IL-1 RA antibodies, demonstrating that the inhibitory effects of SGG on T-helper cytokine production are dependent on IL-1 RA (Figure 3B).

### Soluble galactosaminogalactan induces Il-1Ra in vivo and IL-1 RA increases susceptibility to aspergillosis.

The in vitro stimulations described above suggest that the immunomodulatory effects of SGG are due to inhibition of IL-1 bioactivity by inducing IL-1 RA. To assess whether this has relevant consequences in-vivo, we measured IL-1 RA transcription in the lungs of mice infected with Aspergillus with or without the administration of SGG. Induction of IL-1 RA was increased in the presence of SGG (Figure 4A). To determine which cells are responsible for the induction of IL-1 RA we isolated macrophages, neutrophils and epithelial cells from the lungs of naive mice. Macrophages and neutrophils, but not epithelial cells, induced IL-1 RA after pre-stimulation with Aspergillus in the presence of SGG (Figure 4B).

To investigate the significance of IL-1 RA in vivo and to determine whether the effects of SGG induced by SGG are dependent on IL-1 RA, we studied the effects of SGG in wild type (WT) and IL-1 RA-/- mice with invasive aspergillosis. IL-1 RA-/- mice were highly resistant to invasive aspergillosis, supporting the important role of this anti-inflammatory molecule during invasive aspergillosis (Figure 4C). In line with previous observations, SGG increased the susceptibility to invasive aspergillosis in WT mice (Figure 4C). However, SGG did not result in a higher susceptibility to invasive aspergillosis in IL-1 RA-/- mice (Figure 4C). Lung homogenates of Aspergillus-infected mice that received SGG contained higher concentrations of IL-1 RA protein compared to Aspergillus infected mice that did not receive SGG (Figure 4D). These data demonstrate that IL-1 RA has an important role in invasive aspergillosis, and support the concept that the induction of IL-1 RA by SGG has important clinical consequences.

### Induction of IL-1 RA by SGG is dependent on Syk and TLR3/TRIF.

Since the pattern recognition receptors that recognize SGG remain to be elucidated, we investigated which PRR is important for the induction of IL-1 RA by SGG in human PBMCs. Blocking dectin-1 or complement receptor 3 (CR3) did not alter IL-1 RA production (Figure 5A). In addition, blocking the Toll like receptor (TLR) pathways TLR2 and TLR4, which are known to be involved in recognizing Aspergillus PAMPs (Bellocchio et al., 2004a; Bellocchio et al., 2004b; Bochud et al., 2008; Mambula et al., 2002; Meier et al., 2003), did not result in a significant reduction of IL-1 RA (Figure 5B). In contrast, inhibition of Syk demonstrated a slight, yet significant, decrease in IL-1 RA induction induced by SGG, suggesting that the recognition receptor(s) involved in recognizing SGG in human cells are partially dependent on Syk signalling (Figure 5C).

To identify the PRR pathways that are involved in the capacity of SGG to increase the susceptibility to pulmonary aspergillosis, WT, TLR2-/-, TLR3-/-, TLR4-/-, TLR6-/- TLR9-/-, TRIF-/- and MyD88-/- mice were infected with Aspergillus with or without SGG administration (Figure 5D). TLR3-/-, TLR9-/- and TRIF-/- mice displayed lower fungal burden in the presence of SGG (Figure 5D). In addition, the induction of IL-1 RA induced by the administration of SGG during invasive aspergillosis was lost in TLR3-/- and TRIF-/- mice (Figure 5E). Therefore, SGG is able to induce IL-1 RA in vivo in a TLR3/TRIF dependent manner.

### Discussion

In the present study SGG has been demonstrated to be a potent anti-inflammatory effects on human immune cells. SGG inhibits proinflammatory T-helper responses induced both by Aspergillus or cytokine combinations. In addition, SGG specifically induces the anti-inflammatory cytokine IL-1 RA, which reduces IL-1 bioactivity, subsequently resulting in attenuated IL-17, IL-22, and IFNγ responses. The reduction in proinflammatory T-helper cytokine production caused by SGG can be restored in the presence of a neutralizing antibody against human IL-1 RA. Furthermore, IL-1 RA-/- mice are highly resistant to invasive Aspergillus infection underlining the significance of IL-1 RA in-vivo during Aspergillus infection. SGG induces IL-1 RA in vivo and the increased susceptibility to invasive aspergillosis due to the administration of SGG observed in wild type mice was lost in IL-1 RA-/- mice. Although we did not identify the receptor(s) that directly recognize SGG, the induction of IL-1 RA in vitro was Syk dependent, and the effects of SGG in vivo were TLR3/TRIF dependent. These data imply that SGG can exert potent anti-inflammatory effects through the induction of IL-1 RA, which can be detrimental for patients with acute invasive aspergillosis.

Observation that SGG has inhibitory effects on the proinflammatory human anti-Aspergillus host responses is in line with a previous study reported by Fontaine and co-workers who described anti-inflammatory effects of SGG in mice (Fontaine et al., 2011). However, the mechanism through which SGG elicits its immunomodulatory effects remained a question. SGG has the capability to inhibit proinflammatory T-helper cytokine production in human cells. The strong inhibition of SGG on IL 1/Il-23 induced IL-17 production was very similar to IL-1 RA effects, and SGG induces IL-1 RA in human PBMCs, without inducing IL 1 receptor agonists, explaining why SGG acts similar to IL-1 RA

IL-1 RA is a potent anti-inflammatory cytokine that can inhibit the activation of the IL 1 pathway by binding to the IL-1R type 1 receptor and prevent recruitment of the IL-1R accessory protein that is needed for signalling. On the one hand, the proinflammatory effects of the IL-1 pathway need to be controlled since they can be detrimental for the host. The importance of IL-1 RA in controlling these IL 1 mediated proinflammatory responses is underlined by a disease called deficiency of IL-1 RA (DIRA), which is characterized by the absence of IL-1 RA, subsequently resulting in severe Th17 mediated inflammation with neutrophil influx in the skin and bones of these patients (Aksentij evich et al., 2009). On the other hand, a deficient IL 1 pathway is also detrimental for the host, since it is an important protective pathway required to fight infection (van de Veerdonk et al., 2011). Thus the IL-1 axis is a potent pro-inflammatory pathway that needs to be tightly regulated, and IL-1 RA is a crucial player in this regulation. Therefore, it is rather surprising that the role of IL-1 RA in invasive fungal infection has not been studied in detail to date. The absence of IL-1 RA completely protects mice from invasive pulmonary aspergillosis, underscoring the importance of the IL-1 pathway in clearing an acute invasive Aspergillus infection. The observation that SGG induces IL-1 RA *in vivo,* makes SGG a potent anti-inflammatory molecule by suppressing the IL 1 pathway that subsequently results in increased susceptibility to invasive aspergillosis.

One of the maj or risk factors that increases susceptibility to invasive aspergillosis is neutropenia (Marr et al., 2002), and neutrophils are crucial for clearing invasive germinating and hyphal forms of Aspergillus infection (Schaffner et al., 1982). SGG has been shown to inhibit neutrophil recruitment to the lung, which is at least partly due to neutrophil apoptosis (Fontaine et al., 2011). In the presence of SGG, IL-1 RA is increased during invasive aspergillosis. Since IL 1 signaling stimulates neutrophil recruitment, the induction of IL-1 RA could also be an explanation of the decreased neutrophil influx in the lungs of mice that received SGG. However, it must be taken into account that in the setting of chronic inflammation with associated neutrophil influx and increased Th17 responses, IL-1 RA probably plays a protective role, due to its significant capability to suppress the IL 1 signaling pathway, and thus even SGG might be protective for the host. This hypothesis is in line with the observation that IL-1 RA-/- mice develop spontaneous destructive arthritis that is IL 1 and Th17 dependent (Koenders et al., 2008). Therefore, the timing of IL-1 RA is of utmost importance to protect the host from infection and overwhelming inflammation.

In addition to the induction of IL-1 RA by SGG in vitro and in vivo, the observations that the inhibitory effects of SGG on the proinflammatory T-helper cytokine response in human PBMCs can be restored in the presence of a neutralizing antibody against human IL-1 RA, and that the increased susceptibility to invasive aspergillosis induced by SGG is not observed in IL-1 RA-/- mice, strengthen the hypothesis that the anti-inflammatory properties of SGG are dependent on IL-1 RA. Therefore, SGG can interact with the host response by inducing IL-1 RA, which on the one hand might be detrimental for the host in the setting of an acute infection, and on the other hand could be beneficial for the host in the setting of chronic inflammation driven by IL 1.

The receptor that recognizes SGG remains to be elucidated. Interestingly, β-glucans also induce IL-1 RA (Poutsiaka et al., 1993), which means that SGG is recognized through the same receptors that bind β -glucans. Dectin-1 or complement receptor 3 (CR3) two receptors described to recognize β -glucans (Brown and Gordon, 2001; Ross et al., 1985), were not identified as receptors for SGG. Although the production of IL-1 RA by human PBMCs was partially dependent on Syk, the pattern recognition receptor(s) for SGG remains unknown. TLR3-/- mice and TRIF-/- mice, but not MyD88-/- mice, did not induce IL-1 RA in response to SGG, demonstrating that the induction of IL-1 RA by SGG in vivo during invasive aspergillosis is dependent on the TLR3/TRIF pathway. The significance of the TLR3/TRIF pathway during invasive aspergillosis is underlined by the observations that TRIF provides protective tolerance due to the induction of IDO resulting in suppression of neutrophilic influx, and TLR3 deficiency results in increased susceptibility to invasive aspergillosis (de luca 2010 Cell microbial, Carvalho 2012). Whether the effects of the TRIF/TLR3 pathway on the increased susceptibility are dependent on IL-1 RA is currently under investigation.

The data presented here bring new questions into light and open opportunities for future research. First, one of the most interesting observations is the complete protection of IL-1 RA knockout mice to invasive pulmonary aspergillosis. This opens new treatment strategies that target IL-1 RA in the setting of an acute invasive fungal infection. Second, the significant induction of IL-1 RA by SGG makes SGG or a derivative structure of SGG a potential treatment compound for IL 1 mediated diseases, such as joint, bone and muscle diseases and even very common inflammatory diseases such as diabetes and gout (Dinarello et al., 2012). Previously, it has been shown that mitogenic stimulation of monocyte derived macrophages and lymphocytes by αCD3/αCD28 coated beads, or recombinant cytokine-induced IL-17 and IFN-γ production is inhibited in the presence of live *A. fumigatus* (Chai et al., 2010b). Although these changes in cytokine responses were attributed to changes in tryptophan and kynurenine, it is tempting to speculate that SGG secretion by live *A. fumigatus* is responsible for the decreased IL-17 production.

In conclusion, our results demonstrate that SGG has potent anti-inflammatory effects in mice and humans that can be explained by the capability of SGG to induce IL-1 RA. This observation helps to explain one of the immune-evasive mechanisms of *A. fumigatus,* and suggests that inhibition of GAG or IL-1 RA might prove a potential therapeutic target in the treatment of acute invasive pulmonary aspergillosis.

### Galactosaminogalactan protects from experimental colitis

The effect of SGG on experimental murine colitis was investigated and the activity of SGG was evaluated comparatively with anakinra, a known inhibitor of IL-1R (Dinarello, 2009; Dinarello et al., 2012).

### Methodology

Colitis was induced in C57BL/6 wild-type (WT) or p47phox-/- mice that recapitulate several of the features of the human Chronic Granulomatous Disease (CGD) (Huang et al., 1998; Romani et al., 2008). Colitis is a severe complication of CGD patients, the management of which is a challenge because standard immunosuppressive therapy increases the risk of infection in already immunocompromised hosts (Huang et al., 2004). Colitis was induced with dextran sulfate sodium (DSS 2.5% in drinking water for 7 days and then allowed to recover by drinking water alone for an additional 7 days) and the effect of SGG (1 mg/kg intraperitoneally for a week) on colonic injury was assessed. The day after the 7-day of rest, mice were killed and tissues were collected for histology, RNA and cytokine analysis. Colonic sections were stained with H&E, and histology was scored. Cytokines were measured by enzyme-linked immunoabsorbent assay (ELISA) and real time-polymerase chain reaction (RT-PCR) on colonic tissues.

### Principal Findings

Administration of SGG significantly (*, P< 0.05, treated vs untreated mice) attenuated DSS-induced colitis in WT and p47phox-/- mice as observed by a significant reduction of the inflammatory colonic pathology (Figure 6A), damage score (Figure 6B) and inflammatory IL-1b production and a significant increase in anti-inflammatory IL-10 production (Figure 1C). The effects was similar to that observed with anakinra, in terms of inflammatory pathology (Figure 7A), damage score (Figure 7B) and cytokine gene expression (Figure 7C).

### Conclusions/Significance

Together, these results clearly demonstrated that intraperitoneally injected SGG is effective in reducing the severity of DSS-induced colitis in patients with autoinflammatory diseases, such as the CGD patients.

## Claims

1. Galactosaminogalactan comprising α1-4 linked galactose and α1-4 linked N-acetylgalactosamine for use in the treatment of at least one inflammatory disease.

2. Galactosaminogalactan comprising α1-4 linked galactose and α1-4 linked N-acetylgalactosamine for use according to claim 1, **characterized in that** said inflammatory disease is an interleukin-1 mediated disease.

3. Galactosaminogalactan comprising α1-4 linked galactose and α1-4 linked N-acetylgalactosamine for use according to claim 1 or 2, **characterized in that** said galactosaminogalactan induces the expression of IL-1 RA

4. Galactosaminogalactan comprising α1-4 linked galactose and α1-4 linked N-acetylgalactosamine for use according to any of claims 1 to 3, **characterized in that** said galactosaminogalactan is obtained from *Aspergillus fumigatus.*

5. Galactosaminogalactan comprising α1-4 linked galactose and α1-4 linked N-acetylgalactosamine for use according to any of claims 1 to 4, said galactosaminogalactan is obtained from *Aspergillus fumigatus* by a process comprising an extraction step by urea.

6. Galactosaminogalactan comprising α1-4 linked galactose and α1-4 linked N-acetylgalactosamine for use according to any of claims 1 to 5, **characterized in that** said galactosaminogalactan is obtained from *Aspergillus fumigatus* by a process comprising the following steps:
a) Culture of an *Aspergillus fumigatus* mycelium;
b) Alcoholic precipitation of the culture obtained from step a);
c) Incubation of the precipitate obtained from step b) in NaCl,
d) Extraction of the insoluble fraction obtained from step c) by urea.

7. Galactosaminogalactan comprising α1-4 linked galactose and α1-4 linked N-acetylgalactosamine for use according to any of claims 1 to 6, **characterized in that** said inflammatory disease is chosen from acute pancreatitis, ALS, Alzheimer's disease, cachexia/anorexia, asthma, atherosclerosis, chronic fatigue syndrome, fever, insulin diabetes, glomerulonephritis, graft versus host rejection, hemohorragic shock, hyperalgesia, inflammatory bowel diseases, inflammatory conditions of a joint, including osteoarthritis, psoriatic arthritis and rheumatoid arthritis, ischemic injury, including cerebral ischemia (e.g., brain injury as a result of trauma, epilepsy, hemorrhage or stroke, each of which may lead to neurodegeneration), lung diseases (e.g., ARDS), multiple myeloma, multiple sclerosis, myelogenous (e.g., AML and CML) and other leukemias, myopathies (e.g., muscle protein metabolism, esp. in sepsis), osteoporosis, Parkinson's disease, pain, pre-term labor, psoriasis, reperfusion injury, septic shock, side effects from radiation therapy, temporal mandibular joint disease, tumor metastasis, or an inflammatory condition resulting from strain, sprain, cartilage damage, trauma, orthopedic surgery, infection.

8. Galactosaminogalactan comprising α1-4 linked galactose and α1-4 linked N-acetylgalactosamine for use according to any of claims 1 to 7, **characterized in that** said inflammatory disease is chosen from inflammatory bowel diseases.

9. Galactosaminogalactan comprising α1-4 linked galactose and α1-4 linked N-acetylgalactosamine for use according to any of claims 1 to 8, **characterized in that** said inflammatory bowel disease is chosen from Crohn's disease or ulcerative colitis.

10. Galactosaminogalactan comprising α1-4 linked galactose and α1-4 linked N-acetylgalactosamine for use according to any of claims 1 to 9, **characterized in that** said inflammatory disease is chosen from rheumatoid arthritis, osteoarthritis, and other inflammatory conditions resulting from strain, sprain, trauma, infection, cartilage damage or orthopedic surgery.

11. Galactosaminogalactan comprising α1-4 linked galactose and α1-4 linked N-acetylgalactosamine for use according to any of claims 1 to 10, **characterized in that** said inflammatory disease is chosen from inflammatory joint disease, multiple sclerosis, leukemia, ischemic injury, or reperfusion injury.

12. A pharmaceutical composition comprising a galactosaminogalactan comprising α1-4 linked galactose and α1-4 linked N-acetylgalactosamine as defined in any of claims 1 to 6, and optionally a pharmaceutically acceptable carrier.

13. Pharmaceutical composition according to claim 12, **characterized in that** it further comprises a second anti-inflammatory compound chosen from non-steroidal anti-inflammatory drugs (NSAIDs), corticosteroids, slow acting anti-rheumatic drugs (SAARDs), or disease-modifying antiarthitric drugs (DMAARDs).

14. Pharmaceutical composition according to claim 12 or 13, for use in the treatment of at least one inflammatory disease.

15. Pharmaceutical composition according to claim 12 or 13 for use according to claim 14, **characterized in that** said inflammatory disease is an interleukin-1 mediated disease.

16. Pharmaceutical composition according to claim 12 or 13 for use according to claim 14 or 15, **characterized in that** said inflammatory disease is chosen from acute pancreatitis, ALS, Alzheimer's disease, cachexia/anorexia, asthma, atherosclerosis, chronic fatigue syndrome, fever, insulin diabetes, glomerulonephritis, graft versus host rejection, hemohorragic shock, hyperalgesia, inflammatory bowel diseases, inflammatory conditions of a joint, including osteoarthritis, psoriatic arthritis and rheumatoid arthritis, ischemic injury, including cerebral ischemia (e.g., brain injury as a result of trauma, epilepsy, hemorrhage or stroke, each of which may lead to neurodegeneration), lung diseases (e.g., ARDS), multiple myeloma, multiple sclerosis, myelogenous (e.g., AML and CML) and other leukemias, myopathies (e.g., muscle protein metabolism, esp. in sepsis), osteoporosis, Parkinson's disease, pain, pre-term labor, psoriasis, reperfusion injury, septic shock, side effects from radiation therapy, temporal mandibular joint disease, tumor metastasis, or an inflammatory condition resulting from strain, sprain, cartilage damage, trauma, orthopedic surgery, infection.
